# EUROPEAN PATENT SPECIFICATION

(11) **EP 3 807 863 B1**
(45) Date of publication and mention of the grant of the patent: **22.07.2026**
(21) Application number: 19734456.7
(22) Date of filing: 12.06.2019
(51) Int. Cl.: G09B 23/30, B65D 47/18, G01F 11/08, B01L 3/02, B65D 47/08, B65D 47/24, G01N 1/38, G01N 33/15

(54) **PROCESS FOR PREPARING BUFFER SOLUTIONS FOR IN VITRO TESTING OF THE SOLUBILITY OF MEDICAMENTS AND KIT FOR TESTING CLINICAL STATES**
VERFAHREN ZUR HERSTELLUNG VON PUFFERLÖSUNGEN FÜR IN-VITRO-PRÜFUNG DER LÖSLICHKEIT VON MEDIKAMENTEN UND KIT ZUR PRÜFUNG VON KLINISCHEN ZUSTÄNDEN
PROCÉDÉ DE PRÉPARATION DE SOLUTIONS TAMPONS POUR LE TEST IN VITRO DE LA SOLUBILITÉ DE MÉDICAMENTS ET KIT POUR LE TEST DES ÉTATS CLINIQUES

(30) Priority: 12.06.2018 GB 201809627
(43) Date of publication of application: 21.04.2021
(73) Proprietor: Biorelevant.com Ltd, London NW3 6BP (GB)
(72) Inventor: LEIGH, Mathew Louis Steven, London NW3 6BP (GB); DOS SANTOS, Vasco Rafael Fernandes, London NW3 6BP (GB); LEIGH, Daryl Louis Van, London NW3 6BP (GB); MCCULLOUGH, Orfhlaith Tess, London NW3 6BP (GB); LEIGH, Steven, London NW3 6BP (GB)
(74) Representative: Stevens Hewlett & Perkins
(86) International application number: PCT/GB2019/051633
(87) International publication number: WO 2019/239133

(56) References cited:
- WO-A1-2013/144374
- WO-A1-2014/117265
- JP-A- 2007 263 632
- US-A- 3 366 284
- US-A- 3 857 423
- US-A1- 2008 067 194
- US-A1- 2016 017 405

## Description

### FIELD OF THE INVENTION

This invention is in the field of buffers suitable for physiologically relevant *in vitro* drug dissolution testing, drug solubility testing and/or drug profiling. More specifically, it concerns a method for preparing a buffer solution suitable for use in physiologically relevant *in vitro* drug dissolution testing, drug solubility testing and/or drug profiling, a pack comprising a container and a concentrate of said buffer solution within the container, and a kit comprising such a pack together with other components useful in such testing and profiling.

### BACKGROUND TO THE INVENTION

Standard buffers, for example those described in the *United States Pharmacopeia* (USP), are widely used in, for example, the preparation of dosage forms and in analytical procedures, such as *in vitro* drug dissolution testing, drug solubility testing and/or drug profiling. The buffers are chemically defined, cover a wide pH range (typically between pH 1.2 and 10), and have known ionic strength and buffer capacity. This invention is particularly concerned with providing buffers focussed on physiologically relevant dissolution media and solubility studies, reflecting the pH levels in gastrointestinal (GI) regions under fasted and fed conditions. The term "physiologically relevant" as used herein therefore refers to pH 1.2 to 8.5, spanning the two extremes of pH values for fasted and fed states; inter and intra subject variations in different locations within the same region in the GI tract.

Choosing a suitable buffer composition depends on the desired targeted pH and the physicochemical properties of the active pharmaceutical compound (API) being tested and the purpose of the test, including for example assessing solubility and dissolution across different pH values, scaling (relative) dissolution rates at a constant pH due to buffer composition and concentration, osmolality, and ion concentration (strength) of the physiologically relevant dissolution media. Buffer capacity is related to the composition and the concentration of the buffer. The greater the concentration, the greater the resistance to a change in pH affecting drug dissolution in the media. Dissolution rate typically depends on buffer concentration even though the pH of the dissolution medium is constant. It is important to select an appropriate buffer composition for (preparing) dissolution media in the physiologically relevant pH range when biological surfactants are also present in the media.

Standard buffers are widely used in *in vitro* drug dissolution testing, drug solubility testing and/or drug profiling. These are either made from scratch or, more often, purchased as such or as a concentrate (for example, x10) or as dry powders, and then diluted to a precise, fixed volume (for example, 1.0 L, 200 ml and 100 ml) at a given pH of from 1.2 to 10 (USP).

Tests for drug dissolution and dissolution rates between pH 1.2 and 8.5 require numerous aliquots (portions) of freshly prepared dissolution media at different pHs and the same pH within this range, in the dissolution vessels (USP 2 method). The total amount of media required and to be prepared depends on the number of replicates (n) in each test, sampling intervals and the tests carried out. For example, for examining drug dissolution (release) in fasted states and/or for food effects, up to 10 samples may be taken from the dissolution vessel containing 900 ml of, for example fasted state simulated intestinal fluid (FaSSIF) and fed state simulated intestinal fluid (FeSSIF) dissolution media, over a period of, for example, 6 hours. If the test in each dissolution medium involves a minimum of n= 3, the number of replicates required is 18 and the amount of media is 16.2 L (18 x 900 ml). For the two media (FaSSIF and FeSSIF) the numbers are doubled to 32.4 L (36 x 900 ml). Additional tests in fasted and fed state gastric media (for example, FaSSGIF and FeSSGIF) which require 500 ml of dissolution media in each dissolution vessel, would mean 18 L (36 x 500 ml) of buffer solutions maintained at a given pH. It therefore makes sense to make up the buffer solutions freshly, at the same and/or different pH, from concentrates as the need arises, to save time and effort.

Concentrates of buffer solutions, which are then diluted to provide the desired buffer solution, are known and commercially available. Typically, these concentrates are provided in containers with necks or mouths configured for pouring. However, turbulence occurs during pouring concentrates, which makes it difficult to reproducibly measure out multiple, smaller portions of concentrate with accuracy, precision and speed and without spillage. Accordingly, much of the emphasis in the prior art to date when seeking to develop those concentrates is on facilitating pouring, and in particular on minimising the turbulence that occurs during pouring. An alternative strategy which has been adopted is to place the concentrates in ampoules, pouches and sachets. Whilst this solves the problem of reproducibility, accuracy and precision associated with pouring, it does not provide flexibility for dispensing varying amounts of concentrate.

Bottles and other containers with measured or metered dispensing means are known. For example, US 2016/0017405 A1 refers to compositions for rapid nucleic acid hybridisation that can be packaged in a bottle which comprises a measuring closure. WO 2015/124844 A1 discloses a device for packaging and dispensing a product having a dosing nozzle; WO 2008/068775 A2 describes a metered drop bottle for dispensing microliter amounts of a liquid in the form of a drop; and WO 2004/013009 A1 relates to a dropper bottle and accessories therefor and which is intended for administering eye drops. WO 2018/218341 A1 describes the use of a pipette in the preparation of a working concentration of a red blood cell lysis buffer. However, none of these teach or suggest the benefit of dispensing multiple and precise amounts of a concentrate for use in *in vitro* drug dissolution, drug solubility and/or drug profiling studies.

There therefore remains a need for new methods for preparing buffers suitable for *in vitro* drug dissolution testing, drug solubility testing and/or drug profiling from concentrates, and in particular methods which are pragmatic, flexible, user-friendly, and allow for desired amounts of concentrate to be measured out reproducibly, with accuracy and precision, and without spillage.

The present invention is concerned with concentrates with specified dilution factors focussed on the preparation of buffer solutions between pH 1.2 to pH 8.5 and associated buffer capacity, for the purpose of physiologically relevant *in vitro* drug dissolution tests, drug solubility tests and/or drug profiling, and wherein biological surfactants are also added to the dissolution media. It is to be understood that "physiologically relevant" relates to pH values between 1.2 and 8.5 wherein the (biorelevant) dissolution media further contain biologically relevant surfactants such as bile salts, phospholipids and digested fat components. The effect of surfactants in the dissolution media in relation to buffer capacity, osmolality and ion concentrations are further considerations.

This invention therefore seeks to provide an improved method to obtain fresh buffers by dilution, in the range between pH 1.2 to 8.5; along with targeted osmolality, buffer capacity and ion concentration, for *in vitro* physiologically relevant dissolution tests, solubility tests and drug profiling. The method provides dissolution media that are labour and time saving to prepare, and allows variable or constant amounts of the undiluted concentrate to be dispensed reproducibly, accurately and precisely, and without spillage.

### BRIEF DESCRIPTION OF THE INVENTION

The present invention provides a practical method for preparing buffers by dilution from concentrates, targeting a physiologically relevant pH range in mammalian species (broadly between pH 1.2 and 8.5) suitable for physiologically relevant *in vitro* drug dissolution testing, drug solubility testing and/or drug pH profiling, conforming to physicochemical parameters typically described in p13/14 in this specification. The invention allows for desired amounts of the concentrate composition (also referred to as dilutable aliquots) to be weighed or otherwise measured out reproducibly, with accuracy and precision, and without spillage. In particular, the concentrates are dispensed dropwise, or in a controlled stream, from a deformable container having an orifice or aperture configured to dispense the concentrate in a dropwise manner or in a controlled stream. By dispensing the concentrate from such a container, it is possible to provide multiple portions (or aliquots) of the concentrate reproducibly, with accuracy and precision, and without spillage.

The present invention therefore provides a method for preparing a buffer solution suitable for physiologically relevant *in vitro* drug dissolution testing, drug solubility testing and/or drug profiling according to claim 1 annexed hereto, the method comprising:
(a) dispensing from a deformable container into a second container a predetermined quantity of a concentrate of the buffer solution, said deformable container having a nozzle within which there is an orifice or aperture configured to dispense the said predetermined quantity of the concentrate in a dropwise manner and/or in a controlled stream, the drops having a mass of between 0.02g and 0.25g, and the deviation of the droplet mass from the mean droplet mass is less than 0.015g, and
(b) diluting the predetermined quantity of the concentrate with a predetermined quantity of a solvent to produce the buffer solution;

wherein the concentrate of the buffer solution comprises one or more buffer agents, step (a) comprises dispensing from 1g to 250g of the concentrate and step (b) comprises diluting the concentrate by a factor of from 3 to 50,
such that the buffer solution prepared has a pH of from 1 to 9 and a buffer capacity of from 0 mM/1/pH to 100 mM/1/pH; and
wherein the concentrate further comprises one or more osmolality adjusting agents or the method further comprises a step of adding one or more osmolality adjusting agents after the dilution of step (b) in order that the buffer solution prepared has an osmolality of from 25 mOsm/kg to 700 mOsm/kg.

The present invention further provides a kit suitable for testing biorelevant, physiological and/or clinical outcomes *in vitro* comprising:
(a) a pack comprising a deformable container having a nozzle within which there is an orifice or aperture configured to dispense the contents of the container in a dropwise manner and/or in a controlled stream, the drops having a mass between 0.02g and 0.25g, and the deviation of the droplet mass from the mean droplet mass is less than 0.015g; and a concentrate of the buffer solution inside the deformable container, the buffer solution being suitable for physiologically relevant *in vitro* drug dissolution testing, drug solubility testing and/or drug profiling according to the method of the invention; and
(b) at least one biological surfactant and/or a chemically derived salt of a fatty acid.

As far as the Applicants are aware, defined concentrates which are, for example, 3 to 40 times concentrated, further containing osmotic agents, and a pack suitable for preparing physiologically relevant dissolution media in the range between pH 1.2 and pH 8.5, and targeted buffer capacity, osmolality, and ion concentration with accuracy and precision have not previously been described.

### BRIEF DESCRIPTION OF THE FIGURES

The present invention will now be described solely by way of example with reference to the accompanying figures in which:
Figure 1 shows an example of a ribbed twist open/close cap nozzle which the deformable container may comprise.
Figure 2 shows an example of a natural smooth polypropylene disc top cap with crab claw seal nozzle which the deformable container may comprise.
Figure 3 shows an example of a natural ribbed push pull cap with bore seal nozzle which the deformable container may comprise.
Figure 4 shows an example of a smooth polypropylene flip open/close cap with expanded polyethylene liner nozzle which the deformable container may comprise.
Figure 5 shows an example of a smooth polypropylene flip top cap and crab claw seal nozzle which the deformable container may comprise.

### DETAILED DESCRIPTION OF THE INVENTION

The invention relates to methods for preparing buffer solutions suitable for physiologically relevant *in vitro* drug dissolution testing, drug solubility testing and/or drug profiling between pH 1.2 and pH 8.5, preferably pH 1.2 to pH 7.5, and more preferably pH 1.6 to pH 6.8. Drug dissolution testing and drug solubility testing typically means testing the dissolution and/or solubility of drug compounds. Drug profiling generally refers to, for example, evaluating physiochemical properties such as solubility and pH sensitivity, drug permeability in Caco-2 cell lines, and biopharmaceutical properties of the drug.

For clarity, physiologically relevant dissolution media in this application, comprise biological surfactants such as bile salts and lecithins in the case of fasted state media (for example, FaSSIF), and additionally digested fat components in the case of fed state media (for example, FeSSIF) wherein the media is buffered between pH 1.2 and pH 8.5 depending on the buffer composition, along with appropriate buffer capacity, osmolality and ion concentration. In comparison, standard USP SIF buffers used for dissolution tests do not contain osmotic agents, typically added salt.

Thus, physiologically relevant dissolution medium is a (buffered) solution which may be used to provide useful information at a constant pH, regarding dissolution and rate, solubility and other properties of a drug compound and drug product. For example, the buffer solution may be used to obtain physiologically relevant (biorelevant) media for example, FaSSIF and FeSSIF media, which mimic the *in vivo* conditions in, for example, intestine under fasted and fed states, and dissolution media which mimic fluids in the stomach and colon. Such dissolution tests are useful to predict the *in vivo* dissolution and solubility of pharmaceutical products for food effects.

Typically, the buffer solution or physiologically relevant dissolution medium is a simulated fasted-state or fed-state intestinal, gastric or colonic fluid. Preferred examples of such solutions include fasted state simulated intestinal fluid (FaSSIF), fed state simulated intestinal fluid (FeSSIF), fasted state simulated gastric fluid (FaSSGF), fed state simulated gastric fluid (FeSSGF), fasted state simulated colonic fluid (FaSSCOF) and fed state simulated colonic fluid (FeSSCOF). In addition, these solutions may specifically simulate the *in vivo* environment in humans or dogs. Preferably, the buffer solutions mimic the *in vivo* environment in humans.

The buffer solution may also be a solution useful for active pharmaceutical drug (API) profiling and designation according to the Biopharmaceutical Classification System (BCS).

In the method and kit according to the invention, the undiluted composition is a concentrate of the buffer solution. Thus, the concentrate is a composition capable of being dispensed dropwise in an amount by weight (for example 100 mg by weight) that, once appropriately diluted, forms a desired buffer solution with the targeted pH (between 1.2 to 8.5) depending on the composition of the buffer components. However, the amount of concentrate and composition (wt. %) determines buffer capacity (for example, between 5 and 50), osmolality (for example, between 25 to 300 mOsm/kg for fasted state and between 150 mOsm/kg to 700 mOsm/kg in the fed state) of the a buffer solution (after dilution) and dissolution media thereof (after addition of biological surfactants) along with the appropriate targeted buffer capacity, osmolality and ion content. The proposed target parameters are suitable for physiologically relevant *in vitro* drug dissolution testing, drug solubility testing and/or drug profiling at the targeted pH.

The concentrate may contain all the components required for the desired eventual buffer solution at a pH between 1.2 and 8.5 (i.e. it is only necessary to add the solvent, for example water, to obtain the buffer solution). After dilution with the solvent, the pH of the resultant buffer solution may be adjusted with either HCl or NaOH, as the case may be, to obtain precisely, the pH value required. Osmolality may also be adjusted by the addition of any of the salts or sugars disclosed herein as suitable components for the buffer solution and/or concentrate. Preferably, all the components are included in the concentrate, but may also be added to the constituted buffer solutions and/or dissolution media in order to provide/adjust buffer capacity, osmolality and ion concentration of the dissolution media at the target pH.

The deformable container is a container that may be deformed when pressure is applied to the exterior surface of the container. Such deformation will decrease the internal volume of the deformable container and increase the internal pressure in the deformable container. This causes the contents of the container to be expelled or discharged through the orifice or aperture. The deformation may be either plastic or elastic deformation. Preferably, the deformation is elastic deformation, i.e. the deformable container is an elastically deformable container, since this allows the deformable container to be re-used.

The deformable container is configured to dispense the concentrate in a dropwise manner. Thus, when an increased pressure of low magnitude is applied to the external surface of the deformable container (for example by squeezing the container), the concentrate is dispensed dropwise. Such dropwise dispensing is generally achieved by the combination of a small orifice or aperture having a longest dimension across the orifice or aperture of from 0.5 mm to 2 mm, in combination with the application of an increased pressure of low magnitude. The longest dimension across the orifice or aperture is typically the longest dimension across the opening of the orifice or aperture.

The orifice or aperture typically has an aspect ratio of from 1 to 5. Preferably the aspect ratio is from 1 to 2, more preferably from 1 to 1.5. In a particularly preferred embodiment the orifice or aperture has a substantially circular opening, i.e. the aspect ratio is 1 or approximately 1.

If a pressure of higher magnitude is applied to the external surface of the deformable container, the concentrate may be expelled in a continuous stream. This has the advantage that the concentrate may be rapidly dispensed without spillage or wastage due to turbulent flow.

It will be readily apparent to the skilled person what magnitude of increased external pressure is required in order to dispense the concentrate in a dropwise manner or a continuous stream. Thus, by utilising pressures of different magnitudes, it is possible to control the rate at which the concentrate is dispensed. Thus, it is possible to dispense the concentrate from the deformable container in a two-stage process. First, a higher magnitude pressure is applied in order to rapidly dispense the majority of the desired amount of concentrate. Second, a lower magnitude pressure is applied in order to accurately dispense the final amount of concentrate in a dropwise manner. In this way, quantities of concentrate may be quickly dispensed with a high degree of accuracy.

The deformable container comprises a nozzle. Using a nozzle is typically advantageous as it allows for the concentrate to adopt Newtonian, rather than turbulent, flow if it is discharged in a continuous stream. The orifice or aperture is present in the nozzle. The nozzle may have a lid and/or seal in order to close the nozzle and prevent concentrate escaping from the deformable container. Use of a nozzle generally allows concentrate to be dispensed with a higher degree of accuracy and reliability.

The nozzle is preferably configured to dispense drops having a mass of from 0.02 g to 0.25 g, and/or drops having a volume of from 0.02 ml to 0.25 ml, more preferably 0.02 g to 0.15 g and/or drops having a volume of from 0.02 ml to 0.15 ml. The skilled person will understand that the relationship between the mass and volume of the concentrate will depend on the concentrate, pressure and temperature.

Preferably, the nozzle is configured to dispense drops having highly consistent mass and/or volume. One way to determine this is shown in Example 1 below. Thus, a number (for example 10) drops may be dispensed from the nozzle. The mass or volume of each drop may be measured. The mean mass or volume of the drops may therefore be calculated. Preferably, the deviation droplet mass from the mean droplet mass is typically less than 0.015 g (wherein accuracy is the deviation from the mean droplet mass over a sample size of 10 droplets at room temperature (18 to 25°C)). More preferably, the deviation droplet mass from the mean droplet mass is less than 0.010 g.

The nozzle may be any type of nozzle suitable for dispensing drops in a reliable and accurate manner. Such nozzle types will be well known to a person skilled in the art. Examples of nozzle types suitable for use in the present invention include the following (i) to (v):
(i) A ribbed twist open close cap nozzle with bore seal, i.e. a nozzle having a ribbed portion, a seal in the bore and which may be opened and closed by twisting the nozzle cap.
(ii) A disc top cap with crab claw seal, preferably a polypropylene disc top cap with crab claw seal i.e. a nozzle having a disc shaped top cap, preferably made of polypropylene, and a crab claw seal.
(iii) A ribbed push pull cap with bore seal, i.e. a nozzle having a ribbed portion, a seal in the bore, and which may be opened and closed by pushing and pulling the nozzle cap.
(iv) A smooth, preferably polypropylene, flip open close cap with expanded, preferably polyethylene, liner, i.e. a nozzle having a smooth portion and a cap which opens and closes via a flip mechanism and is preferably made of polypropylene with an expanded liner which is preferably made of polyethylene.
(v) A smooth polypropylene flip top cap and crab claw seal, preferably a smooth polypropylene flip top cap and crab claw seal, i.e. a nozzle having a smooth portion and a cap which opens and closes via a flip mechanism and has a crab claw seal.

Most preferably, the nozzle is a ribbed twist open close cap nozzle with bore seal or a ribbed push pull cap with bore seal and reduce variability between dispensed drops as shown in Example 1. While the above described nozzles are described as having smooth or ribbed portions, the skilled person will be aware that these portions do not affect the function of the nozzle and any smooth or ribbed portion could be replaced by a portion having a different texture.

The deformable container typically comprises a plastics material. For example, the deformable container may be made of a plastics material, i.e. substantially consists of, or consists of, a plastics material. The nozzle on the deformable container may also comprise, substantially consist of, or consist of, a plastics material. When a nozzle is present, it may comprise a different plastics material to the deformable container.

The plastics material is typically a plastics material selected from high density polyethylene (HDPE), low density polyethylene (LDPE), polypropylene (PP), polypropylene copolymer (PPCO), polymethylpentene (PMP), fluorinated high density polyethylene (FLPE), fluorinated ethylene propylene (FEP), perfluoroalkoxy (PFA), and ethylene tetrafluoroethylene (ETFE), and combinations thereof.

The deformable container may comprise graduations, which facilitate determining the amount of concentrate remaining in the container and determining the amount of concentrate dispensed. The graduations may be on the deformable container itself, for example graduations or markings on the container. Alternatively, the graduations may be on another material that is affixed to the container (for example, on a sticker).

The size of the deformable container is not particularly limited. The internal volume of the deformable container (i.e. the maximum volume of concentrate which will fit inside the deformable container) is typically less than 2.0 L. Preferably, the internal volume is from 5 ml to 1.5 L, more preferably 10 ml to 1.2 L and further preferably 50 ml to 1.0 L. Sizes below 1.0 L are less cumbersome and are more suitable for a pack or in a kit.

Preferably, the container can be held or used, preferably with one hand, for dispensing dropwise or in a controlled stream.

The buffer solutions are typically aqueous solutions. Typically, the buffer solution is made by diluting the concentrate in water, preferably distilled water, deionised water, purified water or a combination thereof. Preferably, the water is deaerated.

It will be apparent to the skilled person that the amount of concentrate dispensed will depend on the desired volume of buffer solution and the concentration factor (i.e. how many times more concentrated the concentrate is compared to the desired buffer solution). The skilled person will readily be able to calculate the necessary amount (mass or volume) of concentrate and dilution factor for any desired buffer solution. Accordingly, the skilled person will readily be able to calculate the required predetermined quantity of concentrate to be dispensed into the second container. Similarly, the skilled person will readily be able to calculate the required predetermined quantity of solvent to use to dilute the concentrate to produce the buffer solution.

The compositions within the container are for targeting a specific pH value for physiologically relevant dissolution media within the biologically relevant pH range from pH 1.2 to 8.5 found in fasted and fed state GI fluids. The dissolution profile of drugs (for example, charged Class 1 and 3 compounds (BCS classification) are necessarily tested across the said pH range to understand the effects at the same, constant pH value in relation to buffer concentration (buffer capacity), electrolyte content (ionic concentration), osmolality in order to choose the most appropriate pH and pH composition for examining dissolution and solubility rates under same and different pH conditions.

Total buffer concentration and buffer capacity are parameters that can be used to influence the relative dissolution rate.

The amount or volume of concentrate dispensed is typically less than 1.0 kg, and/or less than 1.0 L. Preferably, the volume of concentrate dispensed is from 5 g to 250 g, and/or 5 ml to 250 ml.

The concentrate is preferably as concentrated as possible, so that the minimal mass or volume of concentrate is required for the desired buffer solution. The maximum concentration will be determined by the solubility of the components in the concentrate. The concentrate is typically from 2 times to 100 times more concentrated than the buffer solution. Preferably, the concentrate is from 3 to 50 times more concentrated, more preferably 10 times to 50 times more concentrated, further preferably 20 times to 40 times more concentrated, further preferably still 25 times to 35 times more concentrated. The solvent used in the concentrate is typically water, preferably distilled water, deionised water, purified water or a combination thereof. Preferably, the water is deaerated.

The amount of buffer solution (i.e. an "aliquot") prepared by the method of the invention is typically from 10 ml to 30 L, preferably 500 ml to 10 L, more preferably 100 ml to 1 L. The volume/amount per vessel used for dissolution testing is between 300 ml to 1L. Typically, 500 to 900 ml with repeats of between n=3 to n=12.

The buffer solution typically has a pH of from 1.2 to 8.5. pH is an important parameter in dissolution tests, because the pH of the medium influences drug solubility and dissolution rate, particularly for ionisable compounds designated BCS Class 1 and 3. Thus, buffers for pH control of the media are necessary. Without pH stability, the reliability and reproducibility of the results are compromised, particularly for charged, acidic and basic compounds. Buffer capacity provides resistance to a change in pH affecting relative drug dissolution rates.

Preferred buffers for use in this invention for preparing physiologically relevant dissolution media along with targeted osmolality, buffer capacity and ion content are chosen from, for example, acetate, citrate and phosphate buffer concentrates and combinations thereof.

The pH of the buffer solution is from 1.0 to 9.0, preferably 1.2 to 8.5.

The resultant buffer solution typically has a buffer capacity of 0 mM/l/pH to 100 mM/l/pH, preferably 1 mM/1/pH to 50 mM/l/pH, more preferably 2 mM/1/pH to 20 mM/l/pH.

The resultant buffer solution typically has an osmolality of 25 mOsm/kg to 700 mOsm/kg, preferably 100 mOsm/kg to 400 mOsm/kg.

The resultant buffer solution typically has a surface tension of below 100 mN/m, preferably from 30 mN/m to 90 mN/m.

The properties of the buffer solution will depend on the purpose of the buffer solution, for example, evaluating food effects and location within the gastrointestinal tract. Preferred diluted buffer solutions include those having:
(a) a pH of 1.2 to 6.0, a buffer capacity of up to 10 mM and an osmolality of from 25 to 140 mOsm/kg;
(b) a pH of from 2.2 to 7.0, a buffer capacity of from 10 to 80 mM and an osmolality of from 50 to 700 mOsm/kg, typically from a pH of 3.0 to 6.5 and osmolality of from 75 to 650 mOsm/kg;
(c) a pH of from 3.0 to 8.5, a buffer capacity of from 5 to 25 mM and an osmolality of from 50 to 400 mOsm/kg, typically from a pH of 4.5 to 8.0

Typical embodiments of preferred buffer solutions that can be prepared using the method of the invention are provided in the Examples below.

Volumes described in the present application refer to volumes measured at 25 °C, unless otherwise specified.

The concentrate and corresponding buffers typically comprise one or more osmolality adjusting agents. The concentrate and corresponding buffers typically comprise one or more inorganic buffer agents. The concentrate and corresponding buffers typically comprise one or more organic buffer agents. Preferably, the concentrate and corresponding buffers comprise one or more osmolality adjusting agents, one or more inorganic buffer agents, or one or more organic buffer agents.

The concentrate and corresponding buffer preferably comprise one or more osmolality adjusting agents selected from sodium chloride, potassium chloride, magnesium chloride, calcium chloride, aluminium chloride, monosaccharides such as glucose or fructose, disaccharides such as sucrose or lactose, a polyol such as glycerol, and carbohydrates such as maltodextrin.

The concentrate(s) and corresponding buffer(s) preferably comprise one or more buffer agents selected from the anhydrous and/or hydrate forms of:
sodium phosphate monobasic;
sodium phosphate dibasic; potassium phosphate monobasic;
potassium phosphate dibasic;
imidazole;
sodium carbonate;
sodium hydrogen carbonate;
sodium cacodylate;
sodium barbital;
hydrochloric acid
sodium hydroxidepotassium hydroxide
acetic acid;
trisodium citrate,
sodium acetate trihydrate
malic acid;
succinic acid;
tripotassium citrate;
maleic acid;
citric acid;
formic acid;
lactic acid;
propionic acid;
2-(N-morpholino)ethanesulfonic acid (MES);
Bis-tris methane (Bis Tris);
2-[(2-amino-2-oxoethyl)-(carboxymethyl)amino]acetic acid (ADA);
N-(2-Acetamido)-2-aminoethanesulfonic acid (ACES);
Bis-tris propane 1,3-bis(tris(hydroxymethyl)methylamino)propane;
piperazine-N,N'-bis(2-ethanesulfonic acid) (PIPES);
2-(carbamoylmethylamino)ethanesulfonic acid (ACES);
2-Hydroxy-3-morpholinopropanesulfonic acid (MOPSO);
Cholamine chloride Cholamine chloride hydrochloride;
3-Morpholinopropane-1-sulfonic acid (MOPS);
N N-bis 2-hydroxyethyl-2-aminoethanesulfonic acid (BES);
2-[[1,3-dihydroxy-2-(hydroxymethyl)propan-2-yl]amino]ethanesulfonic acid (TES);
2-[4-(2-hydroxyethyl)piperazin-1-yl]ethanesulfonic acid (HEPES);
[3-Bis(2-hydroxyethyl) amino - 2-hydroxypropane-1-sulfonic acid] (DIPSO);
[3-Bis(2-hydroxyethyl) amino-2-hydroxypropane-1-sulfonic acid] MOBS;
Acetamidoglycine;
3-[[1,3-dihydroxy-2-(hydroxymethyl)propan-2-yl]amino]-2-hydroxypropane- 1-sulfonic acid (TAPSO);
2,2',2"-Nitrilotri(ethan-1-ol) (TEA);
Piperazine-N,N'-bis(2-hydroxypropanesulfonic acid) (POPSO);
4-(2-Hydroxyethyl)piperazine-1-(2-hydroxypropanesulfonic acid) (HEPPSO);
4-(2-Hydroxyethyl)-1-piperazinepropanesulfonic acid (HEPPS);
N-[Tris(hydroxymethyl)methyl]glycine (Tricine);
tris(hydroxymethyl)aminomethane (Tris);
Glycinamide;
Glycine;
Glycylglycine;
Histidine;
N-(2-Hydroxyethyl)piperazine-N'-(4-butanesulfonic acid) (HEPBS);
2-(Bis(2-hydroxyethyl)amino)acetic acid (Bicine);
[tris(hydroxymethyl)methylamino]propanesulfonic acid (TAPS);
2-Amino-2-Methyl-1-Propanol (AMPB);
2-(Cyclohexylamino)ethanesulfonic acid (CHES);
β-Aminoisobutyl alcohol (AMP);
N-(1,1-Dimethyl-2-hydroxyethyl)-3-amino-2-hydroxypropanesulfonic acid (AMPSO);
3-(Cyclohexylamino)-2-hydroxy-1-propanesulfonic acid, CAPSO Free Acid (CAPSO);
3-(Cyclohexylamino)-1-propanesulfonic acid (CAPS); and
4-(Cyclohexylamino)-1-butanesulfonic acid (CABS).

The osmolality of the buffer solution may be adjusted after dilution by adding one or more osmolality adjusting agents, preferably osmolality adjusting agents as recited above.

A FaSSIF buffer concentrate typically comprises:
up to 6M of salts typically conjugate acid or base, preferably 3M; and
up to 4M of acid or base, preferably 0.8M.

The corresponding diluted FaSSIF buffer prepared from this concentrate typically comprises:
25-200 mM salts, comprising 80-120 mM NaCl and 25-30 mM NaH₂PO₄, preferably about 106 mM NaCl and about 28.4 mM NaH₂PO₄; and
1-50 mM acids.

A diluted FeSSIF buffer concentrate typically comprises:
50-300 mM salts, comprising 180-220 mM NaCl, preferably about 203 mM NaCl; and
10-200 acids, preferably comprising about 144 mM acetic acid.

The invention also provides a kit suitable for testing biorelevant, physiological and/or clinical outcomes *in vitro* comprising a pack comprising a deformable container comprising an orifice or aperture configured to dispense the contents of the container in a dropwise manner and/or in a controlled stream, as herein defined; and (b) a concentrate as herein defined of the buffer solution inside the deformable container. The kit may include other components useful in such testing, such as biological surfactants, preferably bile salts and/or phospholipids and/or fatty acid and their salts and/or products of lipolysis.

The Examples set out below illustrate the invention, and are not to be construed as limiting.

### EXAMPLES

### Example 1 - Testing of nozzles

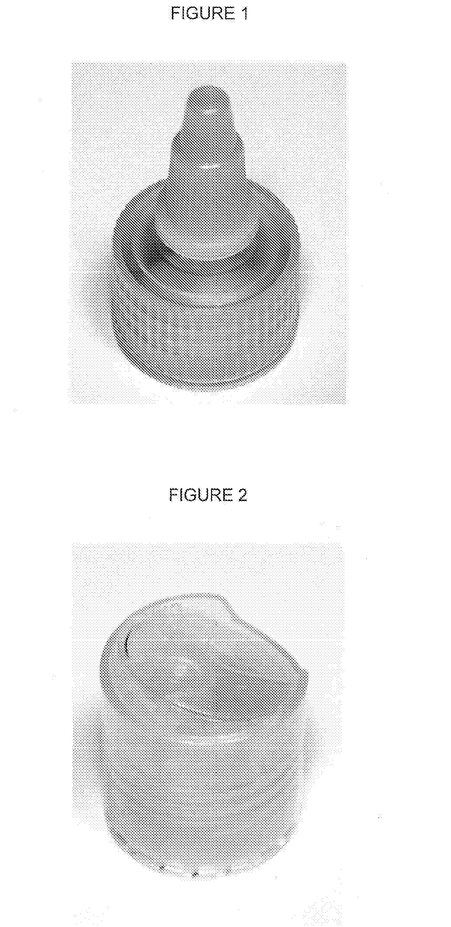

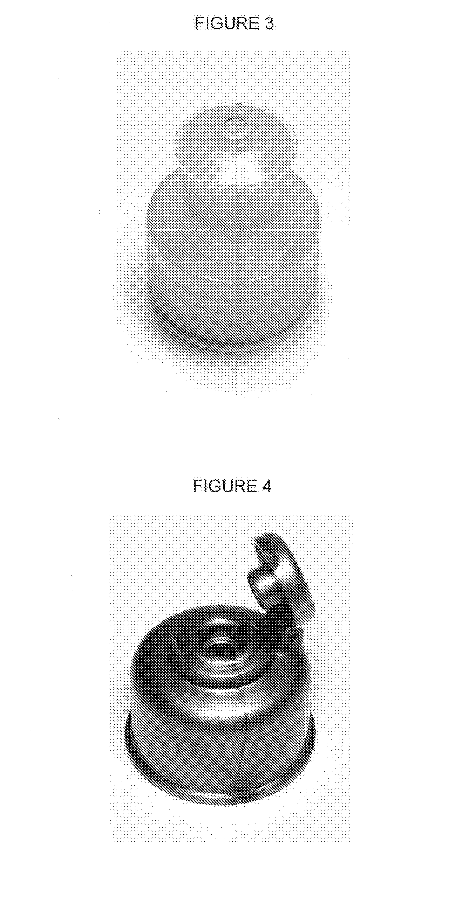

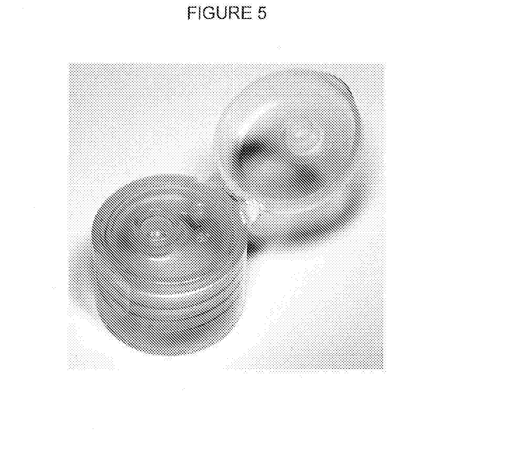

Types were tested for the consistency of the droplet size. The nozzles are shown in Figures 1 to 5. For each nozzle, 10 drops of concentrate (for example, the FaSSIF concentrate of Example 3) were dispensed at room temperature and the mass of each drop was calculated. From this, the mean and standard deviation for each nozzle type was determined. It is desirable that the standard deviation is as low as possible, as this indicates that the droplet size was highly consistent. The results are set out in Tables 1 and 3 to 6 below. Table 2 provides results using the ribbed twist open/close cap nozzle to weigh out target amounts of concentrate required to get a target volume of buffer following dilution and shows the reproducibility of the method.

The data below shows that a ribbed twist open/close cap nozzle and natural ribbed push pull cap with bore seal dispense drops of highly consistent mass.

**Table 1 - Ribbed twist open/close cap nozzle**

| Drop | Mass (g) (room temperature) |
|---|---|
| 1 | 0.067 |
| 2 | 0.065 |
| 3 | 0.068 |
| 4 | 0.059 |
| 5 | 0.060 |
| 6 | 0.067 |
| 7 | 0.065 |
| 8 | 0.073 |
| 9 | 0.066 |
| 10 | 0.070 |
| Range | 0.059 - 0.073 |
| Mean | 0.066 |
| Standard Deviation | 0.0040 |

**Table 2 - Ribbed twist open/close cap nozzle used to weigh out specific weights of concentrate followed by dilution**

| Target Volume (for making up with water) | Amount of aliquot required (target) | Actual amount of aliquot weighed | pH of diluted aliquot solution |
|---|---|---|---|
| 10 ml | 0.32 g | 0.343 g | 6.51 |
| 25 ml | 0.8 g | 0.799 g | 6.51 |
| 25 ml | 0.8 g | 0.844 g | 6.5 |
| 25 ml | 0.8 g | 0.844 g | 6.5 |
| 100 ml | 3.204 g | 3.282 g | 6.5 |
| 100 ml | 3.204 g | 3.353 g | 6.5 |

**Table 3 - Natural smooth PP disc top cap with crab claw seal**

| Drop | Mass (g) (Room temperature) |
|---|---|
| 1 | 0.066 |
| 2 | 0.030 |
| 3 | 0.221 |
| 4 | 0.068 |
| 5 | 0.074 |
| 6 | 0.076 |
| 7 | 0.090 |
| 8 | 0.077 |
| 9 | 0.055 |
| 10 | 0.086 |
| Range | 0.030 - 0.221 |
| Mean | 0.0843 |
| Standard Deviation | 0.0483 |

**Table 4 - Natural ribbed push pull cap with bore seal**

| Drop | Mass (g) (room temperature) |
|---|---|
| 1 | 0.075 |
| 2 | 0.064 |
| 3 | 0.067 |
| 4 | 0.078 |
| 5 | 0.062 |
| 6 | 0.057 |
| 7 | 0.075 |
| 8 | 0.063 |
| 9 | 0.064 |
| 10 | 0.071 |
| Range | 0.057-0.078 |
| Mean | 0.0676 |
| Standard Deviation | 0.0065 |

**Table 5 - Smooth PP flip open/close cap with EPE liner**

| Drop | Mass (g) (Room temperature) |
|---|---|
| 1 | 0.054 |
| 2 | 0.054 |
| 3 | 0.062 |
| 4 | 0.105 |
| 5 | 0.070 |
| 6 | 0.120 |
| 7 | 0.098 |
| 8 | 0.082 |
| 9 | 0.113 |
| 10 | 0.068 |
| Range | 0.054 - 0.113 |
| Mean | 0.0826 |
| Standard Deviation | 0.0234 |

**Table 6 - Smooth PP flip top cap and crab claw seal**

| Drop | Mass (g) (Room temperature) |
|---|---|
| 1 | 0.093 |
| 2 | 0.168 |
| 3 | 0.086 |
| 4 | 0.089 |
| 5 | 0.112 |
| 6 | 0.086 |
| 7 | 0.098 |
| 8 | 0.100 |
| 9 | 0.100 |
| 10 | 0.095 |
| Range | 0.086-0.168 |
| Mean | 0.1027 |
| Standard Deviation | 0.023 |

Examples 2 and 3 show exemplary buffer solutions which can be made according to the process of the invention.

### Example 2 - Methods of making a buffer solution

Buffer concentrates were made in bulk upwards of 5.0 L, e.g., 100 L, in stainless steel vessels. The selected components were accurately weighed and dissolved in purified water or the like, such as demineralised, deionised or distilled water, using a stirrer.

The individual components were quantitatively and qualitatively analysed, including the pH and specific gravity.

100 concentrate portions of 1.0 L were filled into 1 L internal volume deformable containers and plugged, preferably, with bore seal nozzles.

The buffer concentrate could be squeezed out more rapidly via the nozzle in a controlled stream, guided by the calibrations on the outside of the container (where present). The solution was added dropwise towards the end of the target amount. Amounts between 0.02 g to 0.25 g, with surprisingly high accuracy were controlled dropwise (for example using the nozzles of Example 1). The contents of the deformable container were dispensed as multiple aliquots or as a single amount, and the dispensed concentrate was weighed out and apportioned exactly using the deformable container. The preferred range amount of concentrate dispensed was from 5 ml to 250 ml or mass equivalent at 25°C, taking into account the specific gravity (SG) of the buffer concentrate in the container.

Purified water or the like, was added to the accurately weighed or measured aliquots, and depending on the dilution factor, the desired buffer solution at a targeted pH and buffer capacity was obtained.

Buffer capacity may be increased or decreased by the total amount of the aliquot weighed out in respect to the required volume. Osmolality may be adjusted by including a salt or sugar selected from the list of components. Preferably, one or more salts is included.

Further examples according to Examples 3 and 4, to prepare dedicated buffer solutions for making fasted and fed gastric media and media targeting different regions in the gastro intestinal e.g., colonic fluids, may be obtained by selecting suitable components from the disclosure above.

### Example 3 - Fasted State Simulated Intestinal Fluid (FaSSIF) buffer

**Table 7 - Composition of FaSSIF buffer concentrate**

| Component | % weight of component w/v in the FaSSIF concentrate |
|---|---|
| NaCl | 19.190 |
| NaH₂PO₄ · 2H₂O | 13.857 |
| NaOH pellets | 1.302 |
| Water (demineralised water or the like, such as distilled water, deionised water, purified water, etc) | Add up to 100 ml to make 34.3% w/v |

| | |
|---|---|
| The concentrate has a specific gravity of 1.202. | |

The FaSSIF buffer concentrate was diluted by a factor of 31. 3.23 ml (3.88 g equivalent) of FaSSIF buffer contains 1.108 g of non-water components.

**Table 8 - Composition of FaSSIF buffer**

| Component | % weight of component w/v in FaSSIF Buffer |
|---|---|
| NaCl | 0.620 |
| NaH₂PO₄ · 2H₂O | 0.449 |
| NaOH | 0.042 |
| Water (demineralised water or the | Add up to 100 ml (or wt. equivalent) to make FaSSIF |
| like, such as distilled water, deionised water, purified water, etc) | Buffer |

| Properties of FaSSIF buffer | Value |
|---|---|
| pH | 6.5 ± 0.1 |
| Buffer capacity (mM/l/pH) | About 10 |
| Osmolality (mOsm/kg) | 270 |

### Example 4 - Fed State Simulated Intestinal Fluid (FeSSIF) buffer

**Table 9 - Composition of FeSSIF buffer concentrate**

| Component | % weight of component w/v in the FeSSIF concentrate |
|---|---|
| NaCl | 17.783 |
| NaOH | 6.059 |
| Acetic acid | 12.971 |
| Water (demineralised water or the like, such as distilled water, deionised water, purified water, etc) | Add up to 100 ml to make up 36.81% w/v |

| | |
|---|---|
| The concentrate has a specific gravity of 1.158. | |

The FaSSIF buffer concentrate was diluted by a factor of 15. 6.67 ml (7.72 g equivalent) of FeSSIF buffer contains 2.454 g of non-water components.

**Table 10 - Composition of FeSSIF buffer**

| Component | % weight of component w/v in FeSSIF buffer |
|---|---|
| NaCl | 1.186 |
| NaOH | 0.404 |
| Acetic acid | 0.865 |
| Water (demineralised water or the like, such as distilled water, deionised water, purified water, etc) | Add up to 100 mL (or wt equivalent) to make FeSSIF Buffer (D) |

| Parameter for FeSSIF | Value |
|---|---|
| pH | 5.0 ± 0.1 |
| Buffer capacity (mM/l/pH) | About 75 |
| Osmolality (mOsm/kg) | 670 |

### Example 5 - Fasted State Simulated Gastric Fluid (FaSSGF)

FaSSGF concentrate may be diluted by a factor of 30 according to the method of the invention, to yield FaSSGF buffer at pH 1.6 ± 0.1; Buffer capacity: not buffered; Osmolality 120 mOsm/kg).

### Example 6 - Fasted State Simulated Intestinal Fluid V2 (FaSSIF-V2)

FaSSIF-V2 concentrate may be diluted by a factor of 3 according to the method of the invention, to yield FaSSIF V2 buffer solution at pH 6.5 ± 0.1; Buffer capacity 10mM/L/ΔpH; Osmolality 180 mOsm/kg.

### Example 7 - Fed State Simulated Intestinal Fluid V2 (FeSSIF-V2)

FeSSIF-V2 (maleate) concentrate may be diluted by a factor of 3 according to the method of the invention, to yield FeSSIF V2 buffer at pH 5.8 ± 0.1; Buffer capacity 25 mM/L/ΔpH; Osmolality 390 mOsm/kg).

### Example 8 - Fasted State Simulated Colonic Fluid (FaSSCOF)

FaSSCoF concentrate may be diluted by a factor of 20 according to the method of the invention, to yield FaSSCoF buffer at pH 7.8 ± 0.1; Buffer capacity 16 mM/L/ΔpH; Osmolality 196 mOsm/kg.

### Example 9 - Fed State Simulated Colonic Fluid (FeSSCOF)

FeSSCoF concentrate may be diluted by a factor of 20 according to the method of the invention, to yield FeSSCoF solution at pH 6.0 ± 0.1; Buffer capacity 15 mM/L/ΔpH; Osmolality 207 mOsm/kg).

### Example 10 - Dog Fasted State Simulated Intestinal Fluid (FaSSIF)

Dilutable Dog FaSSIF concentrate may be diluted by a factor of 30 according to the method of the invention, to yield Dog FaSSIF buffer at pH 7.5 ± 0.1; Buffer capacity 13.8 mM/L/ΔpH; Osmolality 181.6 mOsm/kg).

### Example 11 - Preparation of FaSSIF dissolution medium

To the composition of Table 8 (Example 3), 0.224g of FaSSIF/FeSSIF/FaSSGF powder was added to obtain the dissolution medium.

### Example 12 - Preparation of FeSSIF dissolution medium

To the composition of Table 9 (Example 4), 1.120g of FaSSIF/FeSSIF/FaSSGF powder was added to obtain the dissolution medium.

### Example 13 - Two step dilution

A 31 times dilution may be performed using a 2 step dilution wherein an intermediate dilution (e.g. x 2 dilution) is further diluted to achieve the required buffer in the second step.

Buffer concentrates are very convenient and useful when carrying out a "fasted stomach to small intestine" dissolution test. This test can be used to examine how a drug behaves when the formulation passes from the stomach to the small intestine. This experiment is particularly useful for poorly soluble basic drugs and formulations when the drug supersaturates, and tests for precipitation when the stomach contents reach the upper small intestine.

The procedure uses a USP II dissolution apparatus very similar to Method A for extended release products described in Delayed Release Forms - Acid Step USP <711>.

To carry out a "fasted stomach to small intestine dissolution test" dissolution of the dosage form (for example a tablet) is carried out first in FaSSGF pH 1.8 (for example 300 ml) and in a second step a x2.0 concentrate (x2.0) of 300 ml FaSSIF is added to the 300 ml FaSSGF to yield a total volume of 600 ml. This x2.0 FaSSIF concentrate is made by diluting 29.03 ml of x31 concentrate in 300 ml of purified water dissolving 2.016 g of FaSSIF/FeSSIF/FaSSGF powder and making up to 600 ml with the aforementioned 300 ml FaSSGF medium and drug product, thereby yielding the required FaSSIF medium.

## Claims

1. A method for preparing a desired amount of buffer solution suitable for physiologically relevant *in vitro* drug dissolution testing, drug solubility testing and/or drug profiling, the method comprising:
(a) dispensing from a deformable container into a second container a predetermined quantity (aliquot) of a concentrate of the buffer solution, said deformable container having a nozzle within which there is an orifice or aperture configured to dispense the said predetermined quantity of the concentrate in a dropwise manner and/or in a controlled stream, the drops having a mass of between 0.02 g and 0.25 g, and the deviation of the droplet mass from the mean droplet mass is less than 0.015 g, and
(b) diluting the said predetermined quantity of the concentrate with a predetermined quantity of a solvent to produce the desired amount of buffer solution;
wherein the concentrate of the buffer solution comprises one or more buffer agents, step (a) comprises dispensing from 1 g to 250 g of the concentrate and step (b) comprises diluting the concentrate by a factor of from 3 to 50,
such that the buffer solution prepared has a pH of from 1 to 9 and a buffer capacity of from 0 mM/l/pH to 100 mM/l/pH; and
wherein the concentrate further comprises one or more osmolality adjusting agents or the method further comprises a step of adding one or more osmolality adjusting agents after the dilution of step (b) in order that the buffer solution prepared has an osmolality of from 25 mOsm/kg to 700 mOsm/kg.

2. The method according to claim 1, wherein the solvent comprises water, deaerated water, distilled water, deionised water, purified water, or a combination thereof.

3. The method according to claim 1 or claim 2, wherein at least one biological surfactant is added to the buffer solution.

4. The method according to any one of the preceding claims, wherein step (b) comprises diluting the concentrate by a factor of 10 to 50, and more preferably 20 to 40.

5. The method according to claim 1, wherein the one or more osmolality adjusting agents is/are selected from sodium chloride, potassium chloride, magnesium chloride, calcium chloride,aluminium chloride, monosaccharides, disaccharides, polyols, and carbohydrates.

6. The method according to any one of the preceding claims, wherein the one or more buffer agents is/are selected from the anhydrous and/or hydrate forms of:
sodium phosphate monobasic;
sodium phosphate dibasic;
potassium phosphate monobasic;
potassium phosphate dibasic;
imidazole;
sodium carbonate;
sodium hydrogen carbonate;
sodium cacodylate;
sodium barbital;
hydrochloric acid;
sodium hydroxide;
potassium hydroxide;
acetic acid;
trisodium citrate,
sodium acetate trihydrate;
malic acid;
succinic acid;
tripotassium citrate;
maleic acid;
citric acid;
formic acid;
lactic acid;
propionic acid;
2-(N-morpholino)ethanesulfonic acid (MES);
Bis-tris methane (Bis Tris);
2-[(2-amino-2-oxoethyl)-(carboxymethyl)amino]acetic acid (ADA);
N-(2-Acetamido)-2-aminoethanesulfonic acid (ACES);
Bis-tris propane 1,3-bis(tris(hydroxymethyl)methylamino)propane;
piperazine-N,N'-bis(2-ethanesulfonic acid) (PIPES);
2-(carbamoylmethylamino)ethanesulfonic acid (ACES);
2-Hydroxy-3-morpholinopropanesulfonic acid (MOPSO);
Cholamine chloride Cholamine chloride hydrochloride;
3-Morpholinopropane-1-sulfonic acid (MOPS);
N N-bis 2-hydroxyethyl-2-aminoethanesulfonic acid (BES);
2-[[1,3-dihydroxy-2-(hydroxymethyl)propan-2-yl]amino]ethanesulfonic acid (TES);
2-[4-(2-hydroxyethyl)piperazin-1-yl]ethanesulfonic acid (HEPES);
[3-Bis(2-hydroxyethyl) amino - 2-hydroxypropane-1-sulfonic acid] (DIPSO);
[3-Bis(2-hydroxyethyl) amino-2-hydroxypropane-1-sulfonic acid] MOBS;
Acetamidoglycine;
3-[[1,3-dihydroxy-2-(hydroxymethyl)propan-2-yl]amino]-2-hydroxypropane-1-sulfonic acid (TAPSO);
2,2',2"-Nitrilotri(ethan-1-ol) (TEA);
Piperazine-N,N'-bis(2-hydroxypropanesulfonic acid) (POPSO);
4-(2-Hydroxyethyl)piperazine-1-(2-hydroxypropanesulfonic acid) (HEPPSO);
4-(2-Hydroxyethyl)-1-piperazinepropanesulfonic acid (HEPPS);
N-[Tris(hydroxymethyl)methyl]glycine (Tricine);
tris(hydroxymethyl)aminomethane (Tris);
Glycinamide;
Glycine;
Glycylglycine;
Histidine;
N-(2-Hydroxyethyl)piperazine-N'-(4-butanesulfonic acid) (HEPBS);
2-(Bis(2-hydroxyethyl)amino)acetic acid (Bicine);
[tris(hydroxymethyl)methylamino]propanesulfonic acid (TAPS);
2-Amino-2-Methyl-1-Propanol (AMPB);
2-(Cyclohexylamino)ethanesulfonic acid (CHES);
β-Aminoisobutyl alcohol (AMP);
N-(1,1-Dimethyl-2-hydroxyethyl)-3-amino-2-hydroxypropanesulfonic acid (AMPSO);
3-(Cyclohexylamino)-2-hydroxy-1-propanesulfonic acid, CAPSO Free Acid (CAPSO);
3-(Cyclohexylamino)-1-propanesulfonic acid (CAPS); and
4-(Cyclohexylamino)-1-butanesulfonic acid (CABS).

7. The method according to any one of the preceding claims, wherein the deformable container has an internal volume of less than 2.0 L, preferably 5 ml to 1.5 L, more preferably 10 ml to 1.2 L, still further preferably 50 ml to 1.0 L.

8. The method according to any one of the preceding claims, wherein the pH of the buffer solution is from 1.2 to 8.5.

9. A kit suitable for testing biorelevant, physiological and/or clinical outcomes *in vitro* comprising:
a pack comprising:
(a) a deformable container having a nozzle within which there is an orifice or aperture configured to dispense the contents of the container in a dropwise manner and/or in a controlled stream, the drops having a mass of between 0.02 g and 0.25 g, and the deviation of the droplet mass from the mean droplet mass is less than 0.015 g; and a concentrate of a buffer solution inside the deformable container, the buffer solution being suitable for physiologically relevant *in vitro* drug dissolution testing, drug solubility testing and/or drug profiling according to the method of any one of claims 1 to 8; and
(b) at least one biological surfactant and/or a chemically derived salt of a fatty acid.

10. The kit according to claim 9, wherein the nozzle of the deformable container is:
(i) a ribbed twist open close cap nozzle with bore seal;
(ii) a polypropylene disc top cap with crab claw seal;
(iii) a ribbed push pull cap with bore seal;
(iv) a smooth polypropylene flip open close cap with expanded polyethylene liner; or
(v) a smooth polypropylene flip top cap and crab claw seal.

11. The kit according to claim 9 or claim 10, wherein the biological surfactant comprises bile salts and/or phospholipids and the chemically derived salt of a fatty acid is sodium oleate.

## Patentansprüche

1. Verfahren zur Herstellung einer gewünschten Menge einer Pufferlösung, die zur In-vitro-Prüfung physiologisch relevanter Arzneimittelauflösung, Arzneimittellöslichkeit und/oder Arzneimittelprofilierung geeignet ist, wobei das Verfahren umfasst:
(a) das Abgeben einer vorbestimmten Menge (Aliquot) eines Konzentrats der Pufferlösung aus einem verformbaren Behälter in einen zweiten Behälter, wobei der verformbare Behälter eine Düse aufweist, in der sich eine Öffnung oder ein Durchlass befindet, die bzw. der so ausgebildet ist, dass die vorbestimmte Menge des Konzentrats tropfenweise und/oder in einem kontrollierten Strahl abgegeben wird, wobei die Tropfen eine Masse zwischen 0,02 g und 0,25 g aufweisen und die Abweichung der Tropfenmasse von der mittleren Tropfenmasse weniger als 0,015 g beträgt, und
(b) das Verdünnen der vorbestimmten Menge des Konzentrats mit einer vorbestimmten Menge eines Lösungsmittels, um die gewünschte Menge an Pufferlösung herzustellen;
wobei das Konzentrat der Pufferlösung ein oder mehrere Puffermittel umfasst, Schritt (a) das Abgeben von 1 g bis 250 g des Konzentrats umfasst und Schritt (b) das Verdünnen des Konzentrats um den Faktor 3 bis 50 umfasst,
so dass die hergestellte Pufferlösung einen pH-Wert von 1 bis 9 und eine Pufferkapazität von 0 mM/l/pH bis 100 mM/1/pH aufweist; und
wobei das Konzentrat ferner ein oder mehrere Osmolalitätsregulierungsmittel umfasst oder das Verfahren ferner einen Schritt zum Hinzufügen eines oder mehrerer Osmolalitätsregulierungsmittel nach der Verdünnung von Schritt (b) umfasst, damit die hergestellte Pufferlösung eine Osmolalität von 25 mOsm/kg bis 700 mOsm/kg aufweist.

2. Verfahren nach Anspruch 1, wobei das Lösungsmittel Wasser, entlüftetes Wasser, destilliertes Wasser, entionisiertes Wasser, gereinigtes Wasser oder eine Kombination davon umfasst.

3. Verfahren nach Anspruch 1 oder Anspruch 2, wobei mindestens ein biologisches Tensid zu der Pufferlösung hinzugefügt wird.

4. Verfahren nach einem der vorhergehenden Ansprüche, wobei Schritt (b) das Verdünnen des Konzentrats um den Faktor 10 bis 50, vorzugsweise 20 bis 40, umfasst.

5. Verfahren nach Anspruch 1, wobei das eine oder die mehreren Osmolalitätsregulierungsmittel ausgewählt sind aus Natriumchlorid, Kaliumchlorid, Magnesiumchlorid, Calciumchlorid, Aluminiumchlorid, Monosacchariden, Disacchariden, Polyolen und Kohlenhydraten.

6. Verfahren nach einem der vorhergehenden Ansprüche, wobei das eine oder die mehreren Puffermittel ausgewählt sind aus wasserfreien und/oder hydratisierten Formen von:
einbasischem Natriumphosphat;
zweibasischem Natriumphosphat;
einbasischem Kaliumphosphat;
zweibasischem Kaliumphosphat;
Imidazol;
Natriumcarbonat;
Natriumhydrogencarbonat;
Natriumkacodylat;
Natriumbarbital;
Chorwasserstoffsäure;
Natriumhydroxid;
Kaliumhydroxid;
Essigsäure;
Trinatriumcitrat,
Natriumacetat-Trihydrat;
Apfelsäure;
Bernsteinsäure;
Trinatriumcitrat;
Maleinsäure;
Citronensäure;
Ameisensäure;
Milchsäure;
Propionsäure;
2-(N-Morpholino)ethansulfonsäure (MES);
Bis-Tris-Methan (Bis-Tris);
2-[(2-Amino-2-oxoethyl)-
(carboxymethyl)amino]essigsäure (ADA);
N-(2-Acetamido)-2-aminoethansulfonsäure (ACES);
Bis-Tris-Propan-1,3-
Bis(tris(hydroxymethyl)methylamino)propan;
Piperazin-N,N'-bis(2-ethansulfonsäure) (PIPES);
2-(Carbamoylmethylamino)ethansulfonsäure (ACES);
2-Hydroxy-3-morpholinopropansulfonsäure (MOPSO);
Cholaminchlorid Cholaminchlorid-Hydrochlorid;
3-Morpholinopropan-1-sulfonsäure (MOPS);
N,N-Bis-2-hydroxyethyl-2-aminoethansulfonsäure (BES);
2-[[1,3-Dihydroxy-2-(hydroxymethyl)propan-2- yl]amino]ethansulfonsäure (TES);
2-[4-(2-Hydroxyethyl)piperazin-1-yl]ethansulfonsäure (HEPES);
[3-Bis(2-hydroxyethyl)amino-2-hydroxypropan-1- sulfonsäure] (DIPSO);
[3-Bis(2-hydroxyethyl)amino-2-hydroxypropan-1-sulfonsäure] MOBS;
Acetamidoglycin;
3-[[1,3-Dihydroxy-2-(hydroxymethyl)propan-2-yl]amino]- 2-hydroxypropan-1-sulfonsäure (TAPSO);
2,2',2''-Nitrilotri(ethan-1-ol) (TEA);
Piperazin-N,N'-bis(2-hydroxypropansulfonsäure) (POPSO);
4-(2-Hydroxyethyl)piperazin-1-(2-
hydroxypropansulfonsäure) (HEPPSO);
4-(2-Hydroxyethyl)-1-piperazinpropansulfonsäure (HEPPS);
N-[Tris(hydroxymethyl)methyl]glycin (Tricin);
Tris(hydroxymethyl)aminomethan (Tris);
Glycinamid;
Glycin;
Glycylglycin;
Histidin;
N-(2-Hydroxyethyl)piperazin-N'-(4-butansulfonsäure) (HEPBS);
2-(Bis(2-hydroxyethyl)amino)essigsäure (Bicin);
[Tris(hydroxymethyl)methylamino]propansulfonsäure (TAPS);
2-Amino-2-methyl-1-propanol (AMPB);
2-(Cyclohexylamino)ethansulfonsäure (CHES);
β-Aminoisobutylalkohol (AMP);
N-(1,1-Dimethyl-2-hydroxyethyl)-3-amino-2-hydroxypropansulfonsäure (AMPSO);
3-(Cyclohexylamino)-2-hydroxy-1-propansulfonsäure,
CAPSO freie Säure (CAPSO);
3-(Cyclohexylamino)-1-propansulfonsäure (CAPS); und
4-(Cyclohexylamino)-1-butansulfonsäure (CABS).

7. Verfahren nach einem der vorhergehenden Ansprüche, wobei der verformbare Behälter ein Innenvolumen von weniger als 2,0 l, vorzugsweise 5 ml bis 1,5 l, vorzugsweise 10 ml bis 1,2 l, am stärksten bevorzugt 50 ml bis 1,0 l aufweist.

8. Verfahren nach einem der vorhergehenden Ansprüche, wobei der pH-Wert der Pufferlösung zwischen 1,2 und 8,5 liegt.

9. Kit, das zur *In-vitro-*Prüfung von biorelevanten, physiologischen und/oder klinischen Endpunkten geeignet ist, umfassend:
eine Packung, umfassend:
(a) einen verformbaren Behälter mit einer Düse, in der sich eine Öffnung oder ein Durchlass befindet, die bzw. der so ausgebildet ist, dass der Inhalt des Behälters tropfenweise und/oder in einem kontrollierten Strahl abgegeben wird, wobei die Tropfen eine Masse zwischen 0,02 g und 0,25 g aufweisen und die Abweichung der Tropfenmasse von der mittleren Tropfenmasse weniger als 0,015 g beträgt; und ein Konzentrat einer Pufferlösung im Inneren des verformbaren Behälters, wobei die Pufferlösung zur In-vitro-Prüfung physiologisch relevanter Arzneimittelauflösung, Arzneimittellöslichkeit und/oder Arzneimittelprofilierung gemäß dem Verfahren nach einem der Ansprüche 1 bis 8 geeignet ist; und
(b) mindestens ein biologisches Tensid und/oder ein chemisch hergestelltes Salz einer Fettsäure.

10. Kit nach Anspruch 9, wobei die Düsen-Verschlusskappe des verformbaren Behälters Folgendes ist:
(i) eine gerippte Drehverschlusskappe mit Bohrungsdichtung;
(ii) eine Polypropylen-Scheibenverschlusskappe mit Krabbenklauen-Dichtung;
(iii) eine gerippte Push-Pull-Verschlusskappe mit Bohrungsdichtung;
(iv) eine glatte Polypropylen-Klappverschlusskappe mit einer Einlage aus expandiertem Polyethylen; oder
(v) eine glatte Polypropylen-Klappverschlusskappe mit einer Krabbenklauen-Dichtung.

11. Kit nach Anspruch 9 oder Anspruch 10, wobei das biologische Tensid Gallensalze und/oder Phospholipide umfasst und das chemisch hergestellte Salz einer Fettsäure Natriumoleat ist.

## Revendications

1. Procédé de préparation d'une quantité souhaitée de solution tampon adaptée aux essais in vitro de dissolution de médicaments, aux essais de solubilité de médicaments et/ou au profilage de médicaments dans des conditions pertinentes sur le plan physiologique, le procédé comprenant :
(a) le transfert, à partir d'un récipient déformable vers un second récipient, d'une quantité prédéterminée (aliquote) d'un concentré de la solution tampon, ledit récipient déformable comportant une buse à l'intérieur de laquelle se trouve un orifice ou une ouverture configuré(e) pour distribuer ladite quantité prédéterminée du concentré sous forme de gouttes et/ou en un flux contrôlé, les gouttes ayant une masse comprise entre 0,02 g et 0,25 g, et l'écart de la masse des gouttelettes par rapport à la masse moyenne des gouttelettes est inférieur à 0,015 g, et
(b) la dilution de ladite quantité prédéterminée de concentré avec une quantité prédéterminée d'un solvant pour produire la quantité souhaitée de solution tampon ;
dans lequel le concentré de la solution tampon comprend un ou plusieurs agents tampons, l'étape (a) consiste à distribuer entre 1 g et 250 g du concentré et l'étape (b) consiste à diluer le concentré d'un facteur compris entre 3 et 50,
de telle sorte que la solution tampon préparée présente un pH compris entre 1 et 9 et une capacité tampon comprise entre 0 mM/pH et 100 mm/pH ; et
dans lequel le concentré comprend en outre un ou plusieurs agents d'ajustement de l'osmolalité ou le procédé comprend en outre une étape consistant à ajouter un ou plusieurs agents d'ajustement de l'osmolalité après la dilution de l'étape (b) afin que la solution tampon préparée ait une osmolalité comprise entre 25 mOsm/kg et 700 mOsm/kg.

2. Procédé selon la revendication 1, dans lequel le solvant comprend de l'eau, de l'eau désaérée, de l'eau distillée, de l'eau désionisée, de l'eau purifiée ou une combinaison de celles-ci.

3. Procédé selon la revendication 1 ou la revendication 2, dans lequel au moins un tensioactif biologique est ajouté à la solution tampon.

4. Procédé selon l'une quelconque des revendications précédentes, dans lequel l'étape (b) consiste à diluer le concentré d'un facteur compris entre 10 et 50, et plus préférablement compris entre 20 et 40.

5. Procédé selon la revendication 1, dans lequel le ou les agents d'ajustement de l'osmolalité sont choisis parmi le chlorure de sodium, le chlorure de potassium, le chlorure de magnésium, le chlorure de calcium, le chlorure d'aluminium, les monosaccharides, les disaccharides, les polyols et les glucides.

6. Procédé selon l'une quelconque des revendications précédentes, dans lequel le ou les agents tampons sont choisis parmi les formes anhydres et/ou hydratées des composés suivants :
Phosphate monobasique de sodium ;
Phosphate dibasique de sodium ;
Phosphate monobasique de potassium ;
Phosphate dibasique de potassium ;
Imidazole ;
Carbonate de sodium ;
Hydrogénocarbonate de sodium ;
Cacodylate de sodium ;
Barbital de sodium ;
Acide chlorhydrique ;
Hydroxyde de sodium ;
Hydroxyde de potassium ;
Acide acétique ;
Citrate trisodique ;
Acétate de sodium trihydraté ;
Acide malique ;
Acide succinique ;
Citrate tripotassique ;
Acide maléique ;
Acide citrique ;
Acide formique ;
Acide lactique ;
Acide propionique ;
Acide 2-(N-morpholino)éthanesulfonique (MES) ;
Bis-tris-méthane (Bis Tris) ;
Acide 2-[(2-amino-2-oxoéthyl)-(carboxyméthyl)amino]acétique (ADA) ;
Acide N-(2-acétamido)-2-aminoéthanesulfonique (ACES) ;
Bis-tris-propane 1,3-bis(tris(hydroxyméthyl)méthylamino)propane ;
Pipérazine-N,N'-bis(acide 2-éthanesulfonique) (PIPES) ;
Acide 2-(carbamoylméthylamino)éthanesulfonique (ACES) ;
Acide 2-hydroxy-3-morpholinopropanesulfonique (MOPSO) ;
Chlorure de cholamine chlorhydrate de chlorure de cholamine ;
Acide 3-morpholinopropane-1-sulfonique (MOPS) ;
Acide N,N-bis(2-hydroxyéthyl)-2-aminoéthanesulfonique (BES) ;
Acide 2-[[1,3-dihydroxy-2-(hydroxyméthyl)propan-2-yl]amino]éthanesulfonique (TES) ;
Acide 2-[4-(2-hydroxyéthyl)pipérazin-1-yl]éthanesulfonique (HEPES) ;
Acide 3-bis(2-hydroxyéthyl)amino-2-hydroxypropane-1-sulfonique (DIPSO) ;
Acide 3-bis(2-hydroxyéthyl)amino-2-hydroxypropane-1-sulfonique MOBS ;
Acétamidoglycine ;
Acide 3-[[1,3-dihydroxy-2-(hydroxyméthyl)propan-2-yl]amino]-2-hydroxypropane-1-sulfonique (TAPSO) ;
2,2',2"-Nitrilotri(éthan-1-ol) (TEA) ;
Acide pipérazine-N,N'-bis(2-hydroxypropanesulfonique) (POPSO) ;
Acide 4-(2-hydroxyéthyl)pipérazine-1-(2-hydroxypropanesulfonique) (HEPPSO) ; Acide 4-(2-hydroxyéthyl)-1-pipérazinepropanesulfonique (HEPPS) ;
N-[tris(hydroxyméthyl)méthyl]glycine (tricine) ;
Tris(hydroxyméthyl)aminométhane (Tris) ;
Glycinamide ;
Glycine ;
Glycylglycine ;
Histidine ;
N-(2-hydroxyéthyl)pipérazine-N'-(acide 4-butanesulfonique) (HEPBS) ;
Acide 2-(bis(2-hydroxyéthyl)amino)acétique (Bicine) ;
Acide
[tris(hydroxyméthyl)méthylamino]propanesulfonique (TAPS) ;
2-Amino-2-méthyl-1-propanol (AMPB) ;
Acide 2-(cyclohexylamino)éthanesulfonique (CHES) ;
Alcool β-aminoisobutylique (AMP) ;
Acide N-(1,1-diméthyl-2-hydroxyéthyl)-3-amino-2-hydroxypropanesulfonique (AMPSO) ;
Acide 3-(cyclohexylamino)-2-hydroxy-1-propanesulfonique, acide CAPSO libre (CAPSO) ;
Acide 3-(cyclohexylamino)-1-propanesulfonique (CAPS) ; et
Acide 4-(cyclohexylamino)-1-butanesulfonique (CABS).

7. Procédé selon l'une quelconque des revendications précédentes, dans lequel le récipient déformable présente un volume interne inférieur à 2,0 L, de préférence compris entre 5 mL et 1,5 L, plus préférablement compris entre 10 mL et 1,2 L, et encore plus préférablement compris entre 50 mL et 1,0 L.

8. Procédé selon l'une quelconque des revendications précédentes, dans lequel le pH de la solution tampon est compris entre 1,2 et 8,5.

9. Kit adapté à l'évaluation in vitro de résultats pertinents sur le plan biologique, physiologique et/ou clinique, comprenant :
un emballage comprenant :
(a) un récipient déformable muni d'une buse à l'intérieur de laquelle se trouve un orifice ou une ouverture configuré(e) pour distribuer le contenu du récipient sous forme de gouttes et/ou en un flux contrôlé, les gouttes ayant une masse comprise entre 0,02 g et 0,25 g, et l'écart de la masse des gouttelettes par rapport à la masse moyenne des gouttelettes est inférieur à 0,015 g ; et un concentré d'une solution tampon à l'intérieur du récipient déformable, la solution tampon étant adaptée à des essais de dissolution de médicaments in vitro pertinents sur le plan physiologique, à des essais de solubilité de médicaments et/ou au profilage de médicaments selon la méthode de l'une quelconque des revendications 1 à 8 ; et
(b) au moins un tensioactif biologique et/ou un sel d'origine chimique d'un acide gras.

10. Kit selon la revendication 9, dans lequel la buse du récipient déformable est :
(i) une buse à bouchon à visser nervuré avec joint d'étanchéité ;
(ii) un bouchon à disque en polypropylène avec joint en forme de pince de crabe ;
(iii) un bouchon à pousser-tirer nervuré avec joint d'étanchéité ;
(iv) un bouchon à rabat en polypropylène lisse avec doublure en polyéthylène expansé ; ou
(v) un bouchon à rabat en polypropylène lisse et un joint en forme de pince de crabe.

11. Kit selon la revendication 9 ou la revendication 10, dans lequel le tensioactif biologique comprend des sels biliaires et/ou des phospholipides et le sel d'origine chimique d'un acide gras est l'oléate de sodium.
